# EUROPÄISCHE PATENTSCHRIFT

(11) **EP 0 482 133 B1**
(45) Veröffentlichungstag und Bekanntmachung des Hinweises auf die Patenterteilung: **18.09.1996**
(21) Anmeldenummer: 91905089.8
(22) Anmeldetag: 13.03.1991
(51) Int. Cl.: A61K 38/16

(54) **VERWENDUNG VON LACTOFERRIN ZUR BEKÄMPFUNG DER TOXISCHEN WIRKUNG DES ENDOTOXINS**
USE OF LACTOFERRIN TO COUNTER THE TOXIC EFFECTS OF ENDOTOXINS
UTILISATION DE LA LACTOFERRINE POUR COMBATTRE L'EFFET TOXIQUE DE L'ENDOTOXINE

(30) Priorität: 14.03.1990 DE 4008033
(43) Veröffentlichungstag der Anmeldung: 29.04.1992
(73) Patentinhaber: NITSCHE, Dietrich, D-24105 Kiel (DE)
(72) Erfinder: NITSCHE, Dietrich, D-24105 Kiel (DE)
(74) Vertreter: Tönnies, Jan G., Dipl.-Ing.
(86) Internationale Anmeldenummer: DE9100214
(87) Internationale Veröffentlichungsnummer: WO9113629

(56) Entgegenhaltungen:
- EP-A- 0 385 118
- Biological Abstracts, Band 88, Nr 6, 1989 G. Sawatzki et al.. "Lactoferrin stimulates colony stimulating factor production in vitro and in vivo."
- Chemical Abstracts, Band 109, 1988 (Columbus, Ohio, US) siehe Seite 325
- Chemical Abstracts, Band 109, 1988 (Columbus, Ohio, US) siehe Seite 378

## Beschreibung

Die Erfindung betrifft die Verwendung von Lactoferrin als Therapeutikum zum Zwecke der Prophylaye und Bekämpfung der toxischen Wirkung des Endotoxins.

Lactoferrin ist ein Glykoprotein mit einem durchschnittlichen Molekulargewicht von 77 000 D. Es kann reversibel zwei Moleküle Eisen binden. Es ist jedoch auch in der Lage, anstatt Eisen andere zwei oder dreiwertige Metallionen anzulagern, wobei u.a. Kupfer, Magnesium, Zink, Mangan und Kobalt infrage kommen.

Erstmals wurde Lactoferrin 1960 aus der Milch isoliert. Der Lactoferringehalt der Muttermilch beträgt ca. 1 mg/dl. Weiterhin wurde Lactoferrin im Blut, sowie in den Granula der neutrophilen Granulocyten, in der Tränenflüssigkeit, im Magensaft und in den Sekreten des Intestinaltraktes nachgewiesen. Im Blut wurde Lactoferrin in einer Konzentration von 40 - 200 µg/dl gefunden.

Im Blut hat Lactoferrin, das hier vorwiegend aus den Granula der neutrophilen Granulocyten freigesetzt wird, besonders im Rahmen von Infektionen oder Entzündungen eine eine gewisse Bedeutung für den Transport von Eisen zu den Zellen des RES [ Van Snick JL, Masson L, Heremans JE. The involvement of lactoferrin in the hyposideremia of acute inflamation. J Exp Med 1974; 140: 1068-1084 ]. Als Folge der raschen Freisetzung von Lactoferrin aus den Granulocyten fand man im Tierexperiment bei Gabe von Bakterien oder Endotoxin eine enge zeitliche Beziehung zur Endotoxingabe und zur Sepsis, weshalb der Anstieg der Lactoferrinkonzentration im Blut als früher Indikator für eine Endotoxinämie oder Septikämie angesehen wurde (Gutteberg TJ., Rokke O., Joergensen T.; Andersen O.,: Lactoferrin as an Indicator of septicemia and Endotoxemia in Pigs. Scand J Infect Dis (1988), 20: 659 - 666).

Eine weitere, wichtige Bedeutung des Lactoferrins, besonders in der Neonatalperiode, wird in der Regulation der Eisenaufnahme aus dem Intestinaltrakt gesehen [ Brock JH, Lactoferrin in human milk: its role in iron absorption and protection against enteric infections in the newborn infant. Arch Dis Child (1980) 55: 417-422 ], da das Lactoferrin der Muttermilch das angelagerte Eisen an die Zellen der Darmmukosa abgeben kann.

Die wichtigste biologische Bedeutung des Lactoferrins wird jedoch in seinem bakteriostatischen Effekt gesehen [ Arnold RR, Brewer M, Gauthier JJ. Bactericidal activity of human Lactoferrin: Sensitivity of a variety of microorganisms. Infection and Immunity (1980) 28: 893-898; Arnold RR, Russel JE, Champion WJ, Gauthier JJ. Bactericidal activity of human Lactoferrin: influence of physical conditions and metabolic state of the target microorganisms. Infection and Immunity (1981) 32: 655-660; Gutteberg T.J., Rokke O., Andersen O., Joergensen T.; Early Fall of Circulating Iron and Rapid Rise of Lactoferrin in Septicemia and Endotoxemia: An Early Defence Mechanism. Scand J Infect Dis, (1989) 21: 709 - 715 ]. Aufgrund seines bakteriostatischen Effektes hat das durch die Muttermilch zugeführte Lactoferrin beim Neugeborenen eine wichtige Rolle als Schutzmechanismus vor Infektionen des Intestinaltraktes. Die bakteriostatische Wirkung beruht auf der hohen Eisenbindungskapazität des Laktoferrins. Eisen ist ein wesentlicher Wachstumsfaktor für die Bakterien. Durch die Komplexbildung des Eisens mit Lactoferrin wird die Konzentration an freiem Eisen in der Umgebung der Bakterien unter dem Wert gehalten, der für das Bakterienwachstum erforderlich ist. Dadurch kommt es zu einer Hemmung des Bakterienwachstums. Mit steigendem Eisengehalt nimmt der bakteriostatische Effekt des Lactoferrins ab. Da im Tierexperiment eisenfreies Lactoferrin ( = Apoform) bei Bakteriämie und Endotoxinämie bereits in der Initialphase der Infektion freigesetzt wird, wurde von Gutteberg et al. davon ausgegangen, daß dem eisenfreien Lactoferrin, aufgrund seines bakteriostatischen Effektes, die Aufgabe eines frühen Abwehrmechanismus des Organismus gegen Bakterien zukommen könnte ( Gutteberg T.J., Rokke O., Andersen O., Joergensen T.; Early Fall of Circulating Iron and Rapid Rise of Lactoferrin in Septicemia and Endotoxemia: An Early Defence Mechanism. Scand. J. Infect. Dis., (1989), 21: 709 - 715 ). Hinweise dafür, daß der Freisetzung der eisenfreien Apoform des Lactoferrins aus Granulocyten bei der Bakteriämie und Endotoxinämie neben der Rolle als Abwehrmechanismus gegen Bakterien auch eine gewisse Rolle als Abwehrmechanismus gegen das Endotoxin zukommen könnte, ergeben sich nicht aus dieser Druckschrift.

Weiterhin scheint Lactoferrin eine gewisse Rolle bei der physiologischen Regulation der Granulopoese zu spielen, wobei diese Funktion noch nicht vollständig aufgeklärt ist. Es wurde beobachtet, daß die Freisetzung des " Colony Stimulating Factor (CSF) " aus den Makrophagen durch Lactoferrin gehemmt werden kann. Dieser Faktor ist für die Granulopoese wichtig. Die Hemmung der CSF-Freisetzung ist vom Eisengehalt des Lactoferrins abhängig [ Broxmeyer HE et al. Identification of lactoferrin as the granulocyte-derived inhibitor of colony-stimulating activity production. J Exp Med 1978; 148: 1052-1068 ].

Endotoxin ist ein Zellwandbestandteil der gramnegativen Bakterien, der erst bei dem Bakterienzerfall freigesetzt wird. Es ist ein Makromolekül mit einem Molekulargewicht bis zu 1 x 10⁶ Dalton, das sich vorwiegend aus Zuckerverbindungen und Fettsäuren zusammensetzt. Daneben kann es noch Proteinreste angelagert haben, die von der Bakterienwand stammen. Das Endotoxinmolekül ist aus drei strukturell und immunbiologisch unterschiedlichen Subregionen aufgebaut:
a) Die Subregion 1, die O-spezifische Kette, besteht aus mehreren, sich wiederholenden Oligosaccharideinheiten, die jeweils von maximal 5 Neutralzuckern gebildet werden. Die Anzahl der Oligosaccharide ist von der Bakterienart abhängig; z.B. hat das hier untersuchte Endotoxin von S. abortus equi in dieser Region 8 Oligosaccharid-Einheiten.
b) Die Subregion 2, das Kernoligosaccharid besteht u.a. aus N-Acetylglucosamin, Glucose, Galactose, Heptose und 2-Keto-3-Desoxyoctonsäure.
c) Die Subregion 3, das Lipid-A ( MG 2000 Dalton ) besteht aus einem phosphorylierten D-Glukosamin-Disaccharid, an das mehrere - ca. 7 - langkettige Fettsäuren in Amid- und Esterbindung geknüpft sind. Der Träger der toxischen Eigenschaften ist das Lipid-A, wobei die toxische Wirkung von einigen Fettsäureresten in dieser Region ausgeht.

Aufgrund der Größe des Endotoxin-Moleküls und aufgrund seiner Ladungsverhältnisse können sich verschiedene Verbindungen und Proteine an den vielen Gruppen bzw. Seitenketten der drei verschiedenen Subregionen des Endotoxins anlagern, ohne dadurch irgendeine Beeinflussung der toxischen Eigenschaften des Endotoxins herbeizuführen. Physiologischerweise ist an das Lipid-A ein Protein gebunden, das sog. Lipid-A-assoziierte Protein. Die Abtrennung dieses Proteinanteils von dem Endotoxin führt bei einigen Endotoxinen zu keinerlei Veränderung der toxischen Wirkung. Bei vielen Endotoxinen wurde jedoch gefunden, daß die Bindung von Proteinen an das Endotoxin eine wesentliche Steigerung der toxischen Wirkung zur Folge haben kann (Rietschel E.TH. et al.(1982) : " Bacterial Endotoxins: Chemical Structure, Biological Activity and Role in Septicaemia " Scand. J. Infect. Dis. Suppl. 31: 8 - 21; S. 17; ).

Endotoxin tritt physiologischerweise auch beim Gesunden aus dem Darm in die Blutbahn über. Die Elimination des Endotoxins aus dem Pfortaderblut erfolgt vorwiegend über die Kupffer'schen Sternzellen der Leber. Zum Schutz vor einer Organschädigung durch das Endotoxin hat das Plasma des Gesunden die Fähigkeit, das ständig aus dem Darm übertretende Endotoxin zu inaktivieren. Der Mechanismus, mit dem dieses erfolgt, ist noch nicht aufgeklärt.

Störungen der Permeabilität im Bereich des Intestinaltraktes, wie sie beispielsweise bei schockbedingten Mikrozirkulationsstörungen, sowie im Rahmen von entzündlichen Darmerkrankungen auftreten, können zu einem gesteigerter Übertritt von Endotoxin aus dem Darmlumen in die Blutbahn führen. Ein vermehrter Übertritt von Endotoxin aus dem Intestinaltrakt in die Blutbahn ist auch dann zu beobachten, wenn es im Rahmen einer enteralen Antibiotikagabe zu einer gesteigerten Endotoxinfreisetzung im Darm kommt [Nitsche D, Stehle M, Hamelmann H. Der Einfluß der Immunsuppression mit Ciclosporin auf die enterogene Endotoxinämie: Tierexperimentelle Untersuchungen. Langenbecks Archiv für Chirurgie 1990, Suppl.]. Ebenso kann es bei ausgedehnten, gramnegativen Infektionen, wie z.B. bei der Peritonitis zu einer starken Endotoxinfreisetzung und damit zu einem vermehrten Endotoxinübertritt von dem septischen Herd in die Blutbahn kommen. Bei einem massiven Endotoxineinstrom erschöpft sich die Fähigkeit des Plasmas zur Inaktivierung des Endotoxins rasch, sodaß vermehrt biologisch aktives Endotoxin im Plasma auftritt, welches dann zu dem klinischen Bild der Endotoxinämie führt. Das Endotoxin bewirkt einerseits die Freisetzung schädigender Mediatoren und führt andererseits zur Störung des Energiestoffwechsels der Zelle, woraus ein Zelluntergang und - bei höherer Endotoxindosis und/oder längerer Endotoxinämiedauer - schließlich ein Organversagen resultieren kann. Aus diesem Grund sind bei allen Erkrankungen, bei denen ein vermehrter Endotoxinübertritt aus dem Darmlumen oder von einem septischen Herd in das Blut zu erwarten ist oder bei denen die physiologische Endotoxinelimination über die Leber gestört ist, zusätzliche therapeutische Maßnahmen erforderlich, mit denen es gelingt, die Endotoxinaktivität im Blut herabzusetzen.

Eine wichtige Zusatzmaßnahme bei der Behandlung der Endotoxinämie ist die Reduzierung des Endotoxineinstroms aus dem Darmlumen, der bereits unter physiologischen Bedingungen ständig vorhanden ist.

Als therapeutische Maßnahme zur Verminderung des Endotoxineinstroms aus dem Darmlumen wurden die orale Zufuhr von Substanzen vorgeschlagen, die in der Lage sind, Endotoxin zu adsorbieren [ Ditter B, Urbaschek R, Urbaschek B. Ability of various adsorbents to bind endotoxins in vitro and to prevent orally induced endotoxemia in mice. Gastroenterology 1983; 84:1547-1552]. Das Hauptproblem besteht bei der Verwendung dieser Substanzen darin, daß hierdurch die Endotoxine im Darmlumen zwar gebunden, jedoch nicht inaktiviert werden, sodaß bei Änderungen des Milieus eventuell wieder Endotoxine freigesetzt werden können.

Wie Studien gezeigt haben, ist eine Herabsetzung der Endotoxinaktivität im Plasma durch Zufuhr von Lipid A-Antikörper möglich ( Ziegler EJ et al; Treatment of gram-negative bacteremia and septic shock with HA-1A human monoclonal antibody against endotoxin. N Eng J Med, 1991:429-436 )
Bis zu einem gewissen Grad kann die Endotoxinaktivität im Plasma auch durch die Gabe polyvalenter Immunglobulin-Präparationen herabgesetzt werden, da bei diesen Präparationen auch ein gewisser Anteil an Lipid A-Antikörpern zu erwarten ist. Mit Ausnahme der Lipid A-Antikörper Präparationen weisen jedoch alle bisher für die Therapie verfügbaren Möglichkeiten entscheidende Nachteile auf, die darin liegen, daß sie nur einen gewissen Prozentsatz der in die Blutbahn übertretenden Endotoxine inaktivieren, sodaß sie bei einem sehr starken Endotoxineinstrom nicht mehr genügend wirksam sind.

Der Erfindung liegt die Aufgabe zugrunde, ein Mittel zu finden, das sich als Therapeutikum für die Bekämpfung der toxischen Wirkung der Endotoxine eignet, indem es in der Lage ist, größere Mengen von Endotoxin im Plasma rasch zu neutralisieren. Es soll jedoch auch in der Lage sein, den zusätzlichen, physiologischen Endotoxineinstrom aus dem Intestinaltrakt in die Blutbahn herabzusetzen, indem es die im Darmlumen freigesetzten Endotoxine direkt im Intestinaltrakt inaktiviert. Hierbei muß es auch möglich sein, daß das Mittel für diesen Zweck per os oder über eine Sonde in den Intestinaltrakt eingebracht werden kann.

Erfindungsgemäß wird diese Aufgabe gelöst durch die Verwendung von Lactoferrin, das ein Metallion angelagert hat. Dieses Lactoferrin kann parenteral appliziert werden oder enteral verabreicht werden. Dabei kann das Lactoferrin Eisen oder aber andere Metallionen gebunden haben. Als sehr wirksam hat sich auch eine Kombination von Lactoferrin mit Immunglobulinen erwiesen, die entweder nur IgG oder IgG und IgM sowie IgA enthalten. Es ist auch möglich, Lactoferrin für die parenterale Applikation in Plasmaderivaten anzureichern, um es mit den darin vorhandenen Proteinen zu kombinieren. Hierbei muß das Lactoferrin für die parenterale Applikation dem humanen Lactoferrin entsprechen. Für die enterale Applikationsform eignet sich auch Lactoferrin tierischen Ursprungs.

Zur Prüfung der Endotoxininaktivierung durch Lactoferrin sowie durch die Kombination von Lactoferrin mit Immunglobulinen wurden folgende Untersuchungen durchgeführt:
Der Einfluß der Metallionen wurde am Beispiel des Eisens und des Zink untersucht. Lactoferrin, humanen und bovinen Ursprungs, in der eisenfreien Apoform und Lactoferrin, welches Eisen bzw. Zink gebunden hat, wurden allein sowie in Kombination mit Immunglobulinpräparationen in vitro und in vivo darauf untersucht, inwieweit sie in der Lage sind, die biologische Aktivität von Endotoxin herabzusetzen.

### Untersuchungen in vitro:

### A) Quantitative Untersuchungen zum Einfluß auf die biologische Aktivität:

Humanes und bovines Lactoferrin und Lactoferrin in Kombination mit Immunglobulinen wurden mit Endotoxin in steigender Konzentration 60 Minuten bei 37°C inkubiert. Nach Abschluß der Inkubation erfolgte die quantitative Bestimmung der biologischen Aktivität des Endotoxins im Überstand mit einer Modifikation des Limulus-Test, unter Verwendung eines chromogenen Substrates ( Nitsche et al. In: Watson, SW, Levin J, Novitsky TJ (eds) DETECTION OF BACTERIAL ENDOTOXINS WITH THE LIMULUS AMEBOCYTE LYSATE TEST. pp 417-429 ( 1987 )). Die untere Nachweisgrenze liegt bei dieser Modifikation des Limulus-Tests bei 2 EU/dl [ 1 EU/dl = 100 pg/dl EC-5 ]. Die mit dem Limulus-Test erfaßte Endotoxinaktivität korreliert mit der biologischen Aktivität des Endotoxins ( Nitsche D, Flad HD und Ulmer A; Endotoxin determination by the Limulus-Test and the biological activity of endotoxin. Does a correlation exist? Publikation in Vorbereitung).

Das humane und bovine Lactoferrin, welche für Untersuchungen verwendet wurde, war weitgehend frei von Eisen ( Lcf.-0̸ ). Hier konnte bei der Eisenbestimmung mit der von Megraw und Bouda ( Megraw RE, Hritz AM, Babson AL und Carroll JJ (1973) Clin. Biochem. 6: 266; Bouda J.(1968) Clin. Chem. Acta 21:159 ) beschriebenen Methode bis zu der unteren Nachweisgrenze, die bei 0,3 µg/dl liegt, in der 5% Lösung kein Eisen nachgewiesen werden. Ebenso konnte mit Hilfe der Flammen-Atomabsorptionsspekspektroskopie in dem Lactoferrin (Lcf.-0̸) kein Zink nachgewiesen werden.
Nach dem Lösen mit 0.9% NaCl wurde das Lactoferrin zunächst sterilfiltriert. Anschließend wurde es mit unterschiedlichem Mengen Eisenchlorid (FeCl₃ ) versetzt, wodurch Lactoferrinlösungen mit unterschiedlichem Eisengehalt erhalten wurden. Für die Untersuchungen wurde Lactoferrin ( Lcf.-Fe(A)) verwendet, dessen Lösung einen Eisengehalt von 28 µg Eisen pro Gramm Lactoferrin hatte, sowie Lactoferrin ( Lcf.-Fe(B) ) mit einem Eisengehalt von 296 µg Eisen pro Gramm Lactoferrin sowie Lactoferrin ( LcF.-Fe(C) ) mit einem Eisengehalt von 1058 µg pro Gramm Lactoferrin.
Weitere Lactoferrinproben (Lcf.-0̸) wurden mit Zinkchlorid(ZnCl₂ ) versetzt, wodurch eine Lactoferrinlösung( LcF-Zn ) mit einem Zinkgehalt von 590̸ µg/Gramm Lactoferrin erreicht wurde.

Folgende Immunglobuline wurden verwendet: 7S-IgG (Intraglobin^{R} , Sandoglobin^{R}) sowie 7S-IgG, angereichert mit 12% IgM ( Pentaglobin^{R} ).

Von folgenden Bakterienstämmen wurden die Endotoxine verwendet: Salmonella abortus equi ( NOVOPYREXAL^{R} ), Pseudomonas aeruginosa Fisher Typ 7, sowie der FDA Endotoxinstandard von E.coli 0113:H1O:KO.

### BESTIMMUNG DER " MITTLEREN PROZENTUALEN INAKTIVIERUNG".

Die prozentuale Inaktivierung der jeweils untersuchten Endotoxinmenge wurde aus der Differenz zwischen der Endotoxinkonzentration, die zugesetzt worden war, und der Endotoxinaktivität bestimmt, die nach der Inkubation mit dem Protein gemessen wurde. Aus den Werten, die auf diese Weise für die verschiedenen Endotoxinkonzentrationen gefunden wurden, wurde für die jeweilige Proteinkonzentration die " mittlere prozentuale Inaktivierung" für den gesamten Endotoxinkonzentrationsbereich (10 EU/dl-1000 EU/dl ) berechnet, der untersucht worden war.

### BESTIMMUNG DER INAKTIVIERUNGSKAPAZITÄT FÜR ENDOTOXIN:

Um die Fähigkeit von Lactoferrin sowie der Kombination von Lacoferrin mit Immunglobulinen zur Inaktivierung von Endotoxin zu quantifizieren, wurde die "INAKTIVIERUNGSKAPAZITÄT" bestimmt. Aus Gründen der Meßgenauigkeit wurde die Bestimmung dieser Größe nicht im Äquivalenzbereich, sondern bei einem Überschuß von 10 EU/dl und 100 EU/dl Endotoxin durchgeführt. Für die Berechnung dieses Parameters wurde diejenige Endotoxinmenge [EU/dl] ermittelt, die der jeweiligen Proteinpräparation zugesetzt werden muß, damit die Konzentration der freien Endotoxine im Überstand nach Inkubation (60 Minuten, 37°C ) mit der Proteinpräparation noch 10 EU/dl bzw. 100 EU/dl beträgt. Diese Endotoxinkonzentration wurde aus der Beziehung berechnet, die zwischen der zugesetzten Endotoxinmenge und der nach der Inkubation im Überstand gemessenen Endotoxinkonzentration für die jeweilige Präparation ( Lactoferrin, Immunglobulin und Kombination von Lactoferrin und Immunglobulin ) gefunden wurde. Zur Bestimmung der Inaktivierungskapazität wurde eine Meßserie durchgeführt, bei der Endotoxin in steigender Konzentration (10 EU/dl, 25 EU/dl, 50 EU/dl, 100 EU/dl, 200 EU/dl, 300 EU/dl, 500 EU/dl sowie 750 EU/dl und 1000 EU/dl) mit den verschiedenen Protein-Präparationen 60̸ Minuten inkubiert wurde. Die Lactoferrinkonzentration im Reaktionsansatz betrug 312,5 mg/dl.

Bei Untersuchung der Kombination von Lactoferrin und Immunglobulin betrug die Konzentration jedes Proteinanteils im Reaktionsansatz 312 mg/dl. Die auf diese Weise ermittelte Endotoxinmenge stellt nach Abzug des vorgegebenen Endotoxinüberschuß von 10 EU/dl bzw. 100 EU/dl die mittlere Endotoxinaktivität [EU/dl] dar, die von der entsprechenden Proteinlösung[mg/dl] unter den jeweiligen Bedingungen inaktiviert werden kann. Bezogen auf eine Gesamtproteinkonzentration von 100 mg/dl stellt diese Endotoxinaktivität ein Maß für die " mittlere Inaktivierungskapazität [ EU/100 mg ] " der jeweiligen Lactoferrin-Präparation bzw. der Kombination von Lactoferrin mit Immunglobulin dar.

### ERGEBNISSE :

### 1. Lactoferrin:

Bei Inkubation von Endotoxin mit Lactoferrin (Lcf.-0̸), welches frei von Eisen ist, findet sich, je nach Art und Konzentration des Endotoxins eine mittlere Abnahme der Endotoxinaktivität von 35,4 % bis 41,2%.

Bei Inkubation von Endotoxin mit Lactoferrin (Lcf.-Fe (A)), welches einen Eisengehalt von 28 µg pro Gramm Lactoferrin hat, kommt es je nach Endotoxinspezies zu einer mittleren Abnahme der Endotoxinaktivität von 62% bis 67,7%.

Bei einem Eisengehalt von 296 µg pro Gramm Lactoferrin ( Lcf.-Fe(B)) wird je nach Endotoxinspezies eine Abnahme der Endotoxinaktivität von 79,1% bis 86,4% erreicht.

Bei einem Eisengehalt von 1058 µg pro Gramm Lactoferrin ( Lcf.-Fe(C)) betrug die Abnahme der Aktivität je nach Endotoxinspezies 89,2% bis 96,7% .

Bei Inkubation von Endotoxin mit Lactoferrin (Lcf.-Zn), welches einen Zinkgehalt von 590 µg pro Gramm Lactoferrin hat, kommt es je nach Endotoxinspezies zu einer mittleren Abnahme der Endotoxinaktivität von 85,2% bis 92,7%.
a) Bezogen auf einen Endotoxinüberschuß von 10 EU/dl beträgt die Inaktivierungskapazität:
   I.) bei Lcf.-0̸ für S.abortus equi 5,8 EU/100 mg, für E.coli 5,2 EU/100mg und für Pseudomonas aeruginosa 4,7 EU/100mg.
   II.) bei Lcf.-Fe(A) für S.abortus equi 48,9 EU/100 mg, für E.coli 57,8 EU/100 mg und für Pseudomonas aeruginosa 17,5 EU/100 mg.
   III.) bei Lcf.-Fe(B) für S.abortus equi 62,1 EU/100 mg, für E. coli 72,3 EU/100 mg und für Pseudomonas aeruginosa 67,7 EU/100 mg.
   IV.) bei Lcf.-Fe(C) für S.abortus equi 97,1 EU/100 mg, für E. coli 126,4 EU/100 mg und für Pseudomonas aeruginosa 152,7 EU/100 mg.
   V.) bei Lcf.-Zn für S.abortus equi 85,6 EU/100 mg, für E. coli 109,6 EU/100 mg und für Pseudomonas aeruginosa 126,8 EU/100 mg.
b) Wird ein Endotoxinüberschuß von 100 EU/dl zugrunde gelegt, dann beträgt die Inaktivierungskapazität:
   I.) bei Lcf.-0̸ für S.abortus equi 20,3 EU/100 mg, für E.coli 18,8 EU/100 mg und für Pseudomonas aeruginosa 32,7 EU/100 mg.
   II.) bei Lcf.-Fe(A) für S.abortus equi 167,9 EU/100 mg, für E.coli 309 EU/100 mg und für Pseudomonas aeruginosa 155,4 EU/100 mg.
   III.) bei Lcf.-Fe(B) für S.abortus equi 233 EU/100 mg, für E.coli 493,2 EU/100 mg und für Pseudomonas aeruginosa 392,4 EU/100 mg.
   IV.) bei Lcf.-Fe(C) für S.abortus equi 376,5 EU/100 mg, für E. coli 598 EU/100 mg und für Pseudomonas aeruginosa 627 EU/100 mg.
   V.) bei Lcf.-Zn für S.abortus equi 312,6 EU/100 mg, für E. coli 527 EU/100 mg und für Pseudomonas aeruginosa 558,8 EU/100 mg.

### 2. Lactoferrin und 7S-IgG

Die Kombination von Lactoferrin mit einer 7S-IgG-Präparation führt zu einer Verbesserung der Inaktivierung von Endotoxin. Von 7S-IgG (312 mg/dl) allein wird die Endotoxinaktivität um 21,5 % bis 33,7% herabgesetzt. Bei Inkubation von Endotoxin mit der Kombination von Lactoferrin ( 312 mg/dl) und 7S-IgG ( 312 mg/dl) wird die Endotoxinaktivität bei Verwendung von Lactoferrin (Lcf.-0̸) welches frei von Eisen ist, bei Endotoxinkonzentrationen unter 200 EU/dl um 58,7 % bis 64,1 % herabgesetzt. Bei höheren Endotoxinkonzentrationen beträgt die Herabsetzung der Endotoxinaktivität unter diesen Bedingungen nur noch 44,3% bis 51,8%. Wird stattdessen eine Kombination mit Lactoferrin (Lcf.-Fe(A)) verwendet, welches einen Eisengehalt von 28 µg Fe³⁺ pro Gramm Lactoferrin hat, dann beträgt die Herabsetzung der Endotoxinaktivität, je nach Endotoxinspezies und Endotoxinkonzentration 72% bis 78,4%.
Bei Kombination von 7S-IgG mit Lactoferrin (Lcf.-Fe(B)), das einen Eisengehalt von 296 µg pro Gramm Lactoferrin hat, wird die Endotoxinaktivität, je nach Endotoxinspezies, um 87,3% bis 92,2% reduziert.
a) Wird ein Endotoxinüberschuß von 10 EU/dl zugrunde gelegt, dann beträgt die Inaktivierungskapazität:
   I.) bei alleiniger Verwendung von 7S-IgG, für S.abortus equi 1,8 EU/100 mg, für E. coli 0,9 EU/100 mg und für Pseudomonas aeruginosa 1,3 EU/100 mg.
   II.) bei Lcf.-0̸ und 7S-IgG für S.abortus equi 9,1 EU/ 100 mg, für E.coli 12,6 EU/100 mg und für Pseudomonas aeruginosa 10,4 EU/100 mg.
   III.) bei Lcf.-Fe(A) und 7S-IgG für S.abortus equi 57,6 EU/100 mg, für E. coli 65,7 EU/100 mg und für Pseudomonas aeruginosa 36,2 EU/100 mg.
   IV.) bei Lcf.-Fe(B) und 7S-IgG für S. abortus equi 77,4 EU/100 mg, für E. coli 86,4 EU/100 mg und für Pseudomonas aeruginosa 82,7 EU/100 mg.
b) Bezogen auf einen Endotoxinüberschuß von 100 EU/dl beträgt die Inaktivierungskapazität:
   I.) bei alleiniger Verwendung von 7S-IgG, für S. abortus equi 19,3 EU/100 mg, für E. coli 9,4 EU/100 mg und für Pseudomonas aeruginosa 16,7 EU/100 mg.
   II.) bei Lcf.-0̸ und 7S-IgG für S. abortus equi 47,6 EU/100 mg, für E. coli 51,9 EU/100 mg und für Pseudomonas aeruginosa 57,3 EU/100 mg.
   III.) bei Lcf.-Fe(A) und 7S-IgG für S. abortus equi 202 EU/100 mg, für E. coli 359 EU/100 mg und für Pseudomonas aeruginosa 282,5 EU/100 mg.
   IV.) bei Lcf.-Fe(B) und 7S-IgG für S. abortus equi 289,7 EU/100 mg, für E. coli 545,2 EU/100 mg und für Pseudomonas aeruginosa 447,1 EU/100 mg.

### 3. Lactoferrin und IgG/A/M (12% IgM)

Bei Zusatz von Lactoferrin zu einer IgG-Präparation, die mit 12% IgM angereichert ist (IgG/A/M), wird eine weitere Steigerung der Inaktivierungskapazität erreicht. Von IgG/A/M (12% IgM) (312 mg/dl) allein wird die Endotoxinaktivität um ca. 45% bis 48% herabgesetzt. Die Abnahme der Endotoxinaktivität beträgt bei Inkubation von Endotoxin mit der Kombination aus IgG/A/M (12% IgM) in einer Konzentration von 312 mg/dl und Lactoferrin, das ebenfalls eine Konzentration von 312 mg/dl hat, bei Verwendung von Lactoferrin (Lcf.-Fe(A)), mit einem Eisengehalt von 28 µg pro Gramm Lactoferrin, je nach Art des Endotoxins 75,9 % bis 81,2%.

Bei Kombination von IgG/A/M mit Lactoferrin (Lcf.-Fe(B)) welches einen Eisengehalt von 296 µg pro Gramm Lactoferrin hat, wird die Aktivität, je nach Endotoxinspezies um 91,8% bis 95,4% herabgesetzt.
a) Bezogen auf einen Endotoxinüberschuß von 10 EU/dl beträgt die Inaktivierungskapazität:
   I.) bei alleiniger Verwendung von IgG/A/M(12% IgM), für S.abortus equi 5,1 EU/100 mg, für E.coli 3,8 EU/100 mg und für Pseudomonas aeruginosa 5,7 EU/100 mg.
   II.) bei Lcf.-Fe(A) und IgG/A/M für S.abortus equi 68,6 EU/100 mg, für E. coli 74,4 EU/100 mg und für Pseudomonas aeruginosa 51,2 EU/100 mg.
   III.) bei Lcf.-Fe(B) und IgG/A/M für S. abortus equi 87,4 EU/100 mg, für E. coli 97,8 EU/100 mg und für Pseudomonas aeruginosa 96,7 EU/100 mg.
b) Für einen Endotoxinüberschuß von 100 EU/dl beträgt die Inaktivierungskapazität:
   I.) bei alleiniger Verwendung von IgG/A/M(12% IgM), für S.abortus equi 48,9 EU/100 mg, für E. coli 35,7 EU/100 mg und für Pseudomonas aeruginosa 67,7 EU/100 mg.
   II.) bei Lcf.-Fe(A) und IgG/A/M für S. abortus equi 268,4 EU/100 mg, für E. coli 419,1 EU/100 mg und für Pseudomonas aeruginosa 394,5 EU/100 mg.
   III.) bei Lcf.-Fe(B) und IgG/A/M für S.abortus equi 383,7 EU/100 mg, für E. coli 629,4 EU/100 mg und für Pseudomonas aeruginosa 586,7 EU/100 mg.

### B) EINFLUSS AUF DIE FREISETZUNG DER MEDIATOREN IL-1 UND IL-6

Als zusätzlicher Parameter für den Einfluß der untersuchten Verbindungen auf die biologische Aktivität des Endotoxins wurde die Freisetzung von IL-1 und IL-6 aus Monocyten herangezogen. Hierzu wurde Endotoxin zunächst mit Lactoferrin bzw. mit der Kombination von Lactoferrin und Immunglobulin inkubiert. Anschließend wurde der Überstand mit Monocyten inkubiert und die Konzentration der aus den Monocyten freigesetzten Mediatoren Interleukin-1 und Interleukin-6 im Überstand bestimmt. Es wurde Lactoferrin ( Lcf.Fe(A) ) verwendet, welches einen Eisengehalt von 28 µg pro Gramm Lactoferrin hat, sowie Lactoferrin ( Lcf.-Fe(B)) mit einem Eisengehalt von 296 µg pro Gramm Lactoferrin.

Folgende Immunglobulinpräparation wurde verwendet: 7S-IgG (IgG/A/M), angereichert mit 12% IgM ( Pentaglobin^{R} )

Von folgenden Bakterienstämmen wurden Endotoxine verwendet: Salmonella abortus equi ( NOVOPYREXAL^{R} ), sowie der FDA Endotoxinstandard EC-5 von E. coli 0113:H1O:KO.

Lactoferrin, Immunglobulin sowie Lactoferrin in Kombination mit Immunglobulin wurden mit Endotoxin 60 Minuten bei 37°C inkubiert. Als Kontrolle wurde Human-Albumin verwendet. Die Konzentration des jeweiligen Proteins im Ansatz betrug: 625 mg/dl, 312 mg/dl und 156 mg/dl. Die Inkubation wurde mit Endotoxinkonzentrationen von 50 EU/dl bis 1500 EU/dl durchgeführt.

Nach der Inkubation wurde die jeweilige Endotoxinlösung mit mononukleären Zellen von gesunden Spendern inkubiert ( 16 Std. bei 37°C ). Anschließend wurde die Konzentration der freigesetzten Mediatoren IL-1 und IL-6 bestimmt. Die Bestimmung von IL-1 erfolgte mit Hilfe des Fibroblasten-Proliferationstest ( Loppnow H; Flad HD, Ulmer AJ et al (1989) "Detection of Interleukin 1 with Human Dermal Fibroblasts. Immunbiol. 179, 283 -291 ), unter Verwendung einer EL4-6.1 Thymomzell-Linie. Die Konzentration von IL-6 wurde ebenfalls über einen Proliferationstest unter Verwendung eines IL-6 abhängigen murinen Hybridoms ( 7TD1 ) bestimmt ( Van Damme J, Cayphas S. et al. (1987) Eur. J.Biochem. 168: 543 -550; sowie Van Oers MHJ, Van der Heyden A, und Aarden LA (1988); Clin. exp. Immunol. 71: 314 -419 ).

Es zeigte sich, daß die Inkubation von Endotoxin mit Lactoferrin zu einer starken Herabsetzung der Freisetzung von Mediatoren aus den mononukleären Zellen durch das Endotoxin führt. Dieser Effekt ist vom Eisengehalt des Lactoferrins abhängig, und zwar derart, daß er durch Erhöhung des Eisengehaltes verbessert werden kann. Durch die Kombination von Lactoferrin mit Immunglobulinen wird die Freisetzung von Mediatoren noch mehr herabgesetzt, wobei auch in der Kombination mit Immunglobulinen eine bessere Wirkung bei höherem Eisengehalt des Lactoferrins erreicht wird.

### Ergebnisse:

### I. Lactoferrin

a) Bei Inkubation von 500 EU/dl Endotoxin mit Lcf.Fe(A) betrug die mittlere Freisetzung von IL-1 bei S.abortus equi 387 U/ml und bei E. coli 229 U/ml. Die Freisetzung von IL-6 betrug bei S.abortus equi 1269 U/ml und bei E. coli 1971 U/ml.
b) Nach Inkubation von Endotoxin ( 500 EU/dl ) mit Lcf.-Fe(B) betrug die mittlere Freisetzung von IL-1 bei S.abortus equi 145 U/ml und bei E. coli 136 U/ml. Die mittlere Freisetzung von IL-6 betrug bei S. abortus 765 U/ml und bei E. coli 831 U/ml.

### II. Lactoferrin und IgG/A/M (12% IgM)

A) Bei Inkubation von 500 EU/dl Endotoxin mit IgG/A/M(12% IgM) allein betrug die mittlere Freisetzung von IL-1 bei S.abortus equi 986 U/ml und bei E. coli 912 U/ml. Die Freisetzung von IL-6 betrug bei S.abortus equi 1802 U/ml und bei E. coli 3387 U/ml.
B) Der Zusatz von Lactoferrin zu der 7S-IgG-Präparation, die mit 12 % IgM angereichert ist ( IgG/A/M ), führte bei einer Immunglobulingesamtkonzentration von 312 mg% und einer Lactoferrinkonzentration, die ebenfalls 312 mg% betrug, nach Inkubation mit 500 EU/dl Endotoxin:
   a) bei Verwendung von Lcf.-Fe(A) zu einer Freisetzung von IL-1 von 251 U/ml bei S. abortus equi und von 167 U/ml bei E. coli. Die IL-6-Menge, die freigesetzt wurde, betrug bei S. abortus equi 920 U/ml und bei E. coli 1268 U/ml.
   b) Bei Kombination von Lcf.-Fe(B) mit IgG/A/M(12%) betrug die mittlere Freisetzung von IL-1 bei S. abortus equi 69 U/ml und bei E. coli 47 U/ml. Die mittlere Freisetzung von IL-6 betrug unter diesen Bedingungen bei S. abortus equi 40̸7 U/ml und bei E. coli 518 U/ml.

### III ALBUMIN

Nach Inkubation von Endotoxin ( 500 EU/dl ) mit Albumin, welches zum Vergleich verwendet wird, beträgt, die mittlere Freisetzung von IL-1 bei Verwendung des Endotoxin von S. abortus equi 2975 U/ml und bei E. coli 2676 U/ml. Die Freisetzung von IL-6 beträgt bei S. abortus equi 30̸18 U/ml und bei E. coli 6233 U/ml.

### C) Einfluß von Lactoferrin auf die Endotoxinaktivität im Plasma

Humanplasma von gesunden Spendern wurde 1:10 verdünnt und durch Erhitzen [ 80°C ] inaktiviert. Anschließend wurde es mit Lactoferrin versetzt, sodaß die Lactoferrinkonzentration des Plasmas 250 mg/dl betrug. Den Kontrollproben wurde die gleiche Menge Albumin zugesetzt. Zu dem mit Lactoferrin bzw. Albumin versetzten Plasma wurde dann Endotoxin ( Pseudomonas-Endotoxin, Coli-Endotoxin ) zugegeben, sodaß im Ansatz eine Endotoxinaktivität von 800 EU/dl und 300 EU/dl erreicht wurde. Nach Endotoxinzugabe wurde der Ansatz dann 60 Minuten bei 37°C inkubiert. Anschließend wurde die Endotoxinaktivität in der Probe bestimmt.

Es zeigte sich, daß die Endotoxinaktivität im Plasma durch den Zusatz von Lactoferrin im Vergleich zu den Proben, denen nur Albumin zugesetzt worden war, signifikant herabgesetzt wird.
a) bei Zusatz von Lcf.-Fe(A) wurde die Endotoxinaktivität im Plasma bei einer Endotoxinkonzentration von 1000 EU/dl um 62,1% ± 4,8% ( n= 18 ) und bei 300 EU/dl Endotoxin um 67,4% ± 5,4% ( n = 16 ) gesenkt.
b) bei Zusatz von Lactoferrin ( Lcf.-Fe(B) ) betrug die Abnahme der Endotoxinaktivität 78,5% ± 6,9% ( n = 18 ) bei 1000 EU/dl Endotoxin und bei 300 EU/dl Endotoxin: 84,1% ± 5,1% ( n = 20 ).

### Zusammenfassende Beurteilung der Ergebnisse:

1. Lactoferrin ist nach Anlagerung von dreiwertigen Metallionen, wie z.B. Eisen, oder auch nach Anlagerung von zweiwertigen Metallionen, wie z.B. Zink in der Lage, konzentrationsabhängig die biologische Aktivität des Endotoxins herabzusetzen.
2. Das Ausmaß, in dem die toxische Wirkung des Endotoxins durch Lactoferrin herabgesetzt wird, nimmt mit der Eisenbeladung des Lactoferrins zu.
   Der Eisengehalt des Lactoferrins sollte hierbei mindestens 0.1 µg pro Gramm Lactoferrin betragen, vorzugsweise jedoch höher als 100 µg pro Gramm Lactoferrin sein.
3. Durch die Kombination von Lactoferrin mit anderen Plasmaproteinen, insbesondere mit Immunglobulinen der IgG- und IgM-Fraktion wird eine signifikant höhere Wirkung auf die biologische Aktivität des Endotoxins erreicht, als sie bei den einzelnen Proteinfraktionen isoliert zu finden ist. Der Synergismus in der Wirkung auf die Endotoxinaktivität führt bei Kombination von Lactoferrin und Immunglobulinen zu einer Potenzierung des inaktivierenden Effektes beider Proteine.
4. Aufgrund des Synergismus zeichnet sich die Kombination von Lactoferrin mit Immunglobulinen durch eine wesentlich größere Inaktivierungskapazität für Endotoxin aus. Auf diese Weise gelingt es, auch ohne eine übermäßig starke Eisenbela stung des Organismus, eine therapeutisch relevante Inaktivierung des Endotoxins zu erreichen.
5. Eine gewisse Herabsetzung der Endotoxinaktivität ist zwar auch bei Kombination von eisenfreiem Lactoferrin mit Immunglobulin möglich, doch um eine größere Inaktivierungska pazität zu erreichen, erweist es sich als günstiger, wenn der Eisengehalt des Lactoferrins auch bei der Kombination mit dem Immunglobulin mindestens 0,1 µg pro Gramm Lactoferrin beträgt. Vorzugsweise sollte der Eisengehalt jedoch höher als 100 µg pro Gramm Lactoferrin sein.
6. Aufgrund seiner Fähigkeit, die biologische Aktivität des Endotoxins herabzusetzen, eignet sich Lactoferrin für die Bekämpfung der toxischen Wirkung von Endotoxin bei Störungen der Endotoxinelimination sowie bei allen krankhaften Veränderungen, bei denen es zu einem gesteigerten Endotoxinübertritt aus dem Intestinaltrakt oder auch von einem septischen Herd in die Blutbahn kommt.
7. Da Lactoferrin auch direkt in den Gastrointestinaltrakt eingebracht werden kann, eignet es sich besonders zur Bekämpfung des Endotoxinübertrittes aus dem Intestinaltrakt. Zu diesem Zweck kann es entweder per os oder über eine Sonde zugeführt werden, und zwar als alleinige Substanz oder in Kombination mit anderen Stoffen, insbesondere mit solchen Stoffen, die der enteralen Ernährung dienen.
8. Aufgrund der größeren Inaktivierungskapazität ist die Kombination von Lactoferrin mit Immunglobulinen für therapeutische Maßnahmen zur Bekämpfung der toxischen Wirkung von Endotoxin bei allen krankhaften Veränderungen geeignet, bei denen es zu einem stärkeren und länger andauernden Endotoxinübertritt aus dem Intestinaltrakt oder von einem septischen Herd in die Blutbahn kommt. Damit ist von Lactoferrin bzw. der Kombination von Lactoferrin mit Immunglobulinen ein günstiger Effekt auf den Verlauf von Endotoxinämien jeglicher Genese zu erwarten.
9. Da Lactoferrin zu einer Hemmung der Freisetzung des für die Granulopoese wichtigen " Colony Stimulating Factors (CSF) " aus den Makrophagen führen kann, sollte bei der intravenösen Applikation von Lactoferrin - je nach Bedarf - zusätzlich " Colony Stimulating Factor " in der rekombinanten oder natürlichen Form substituiert werden.
10̸. Anstelle des Lactoferrin-Eisenkomplex kann auch ein Lactoferrin für die Bekämpfung der toxischen Wirkung des Endotoxins verwendet werden, welches statt Eisen andere Metallionen, wie z.B. Zink, Kupfer, Kobalt, Mangan oder Magnesium angelagert hat.

### Die Erfindung wird durch folgende Beispiele erläutert.

### BEISPIEL: 1

Zur Objektivierung der Bedeutung, den der Eisengehalt des Lactoferrins für dessen Fähigkeit zur Inaktivierung von Endotoxin im Plasma hat, wurden folgende Untersuchungen durchgeführt: Humanplasma von gesunden Spendern wurde mit 0,9% NaCl 1:10 verdünnt und danach durch Erhitzen ( 80°C, 5 Min. ) inaktiviert. Anschließend wurde Lactoferrin zugesetzt, welches einen unterschiedlichen Eisengehalt hatte. Die Lactoferrinkonzentration betrug im Ansatz jeweils 300 mg/dl. Durch Zugabe von unterschiedlichen Mengen FeCl₃ war der Eisengehalt des Lactoferrins so eingestellt worden, daß in der Lösung ein Mol-Verhältnis zwischen Lactoferrin und Eisen von 22 : 1, 1 : 1, 1 : 2,5 und 1: 3,9 bestand.
Nach der Lactoferrinzugabe wurde Endotoxin von Pseudomonas aeruginosa den Plasmaproben in unterschiedlichen Konzentrationen zugesetzt, sodaß im Ansatz Endotoxinkonzentrationen von 50, 100, 200, 300, 500, 750, 1000 und 1500 EU/dl erreicht wurden. Sofort nach der Endotoxinzugabe wurde der Ansatz dann 60 Minuten bei 37°C inkubiert. Danach wurde die Endotoxinrestaktivität bestimmt, die in den Proben noch nachweisbar war. Aus diesen Werten wurde die Inaktivierungskapazität der verschiedenen Lactoferrinlösungen für einen Endotoxinüberschuß von 10 EU/dl berechnet. - Das genaue Vorgehen bei der Berechnung der Inaktivierungskapazität ist in dem Abschnitt " Untersuchungen in vitro " auf S. 12/13 ausführlich beschrieben. -

Für einen Endotoxinüberschuß im Reaktionsansatz von 10 EU/dl wurden in Abhängigkeit von dem jeweiligen Molverhältnis zwischen Lactoferrin und Eisen folgende Werte für die Inaktivierungskapazität von 100 mg/dl Lactoferrin gefunden:
a) 22 : 1 : 17,2 EU pro 100 mg Lactoferrin
b) 1 : 1 : 68,2 EU pro 100 mg Lactoferrin
c) 1 : 2,5 : 179,4 EU pro 100 mg Lactoferrin
d) 1 : 3,9 : 405,7 EU pro 100 mg Lactoferrin

Durch die Erhöhung des Eisenanteils von Lactoferrin gelingt es, die Inaktivierungskapazität zu steigern. Hierbei führt beispielsweise die Erhöhung des Eisengehaltes von einem Molverhältnis zwischen Lactoferrin und Eisen, das 22 : 1 beträgt, auf ein Molverhältnis von 1 : 3,9 zu einer Zunahme der Inaktivierungskapazität um einen Faktor von 23,5. Bei einem Molverhältnis von 1 : 3,9 ist zu erwarten, daß die meisten Lactoferrinmoleküle zwei Moleküle Eisen angelagert haben.

### BEISPIEL: 2

Bei einer Patientin mit einer diffusen Peritonitis, konnte eine erhöhte Endotoxinaktivität im Plasma nachgewiesen werden. Bei Inkubation einer Plasmaprobe dieser Patientin mit einer Immunglobulin-Präparation, die mit IgM-angereichert war, (30 Minuten, 37°C) - Immunglobulinkonzentration im Ansatz: 312 mg/dl - konnte eine Abnahme der Endotoxinaktivität um 16,5% erreicht werden. Bei Inkubation des gleichen Plasmas mit Lactoferrin ( Lcf.-Fe (A)), in einer Konzentration von 312 mg/dl, werden 41% des Endotoxins inaktiviert. Durch Kombination von Lcf.-Fe(A) mit dem Immunglobulin in einer Konzentration von jeweils 312 mg/dl wurde die Endotoxininaktivierung im Plasma der Patientin auf 64 % gesteigert.
Bei Zugabe der gleichen Menge von Lactoferrin (Lcf.-Fe(B)) mit einem höheren Eisengehalt, in entsprechender Konzentration, zum Patientenplasma wurden 63 % der Endotoxine inaktiviert. Bei Kombination dieses Lactoferrins (Lcf.-Fe(B)) mit dem IgM-angereicherten Immunglobulinpräparat(IgG/A/M) wurden sogar 87 % des Endotoxins im Plasma der Patientin inaktiviert. Die gute Senkung der Endotoxinaktivität durch Lactoferrin mit Eisen und die Steigerung des Effektes durch die Kombination von eisenhaltigem Lactoferrin mit Immunglobulinen geben bei Verwendung von Lactoferrin (human) und intravenöser Applikation die Möglichkeit, Endotoxinämien jeglicher Genese zu behandeln. Wegen einer möglichen Hemmung der Freisetzung des " Colony Stimulating Factors " durch Lactoferrin, sollte evtl. bei der intravenösen Applikation von Lactoferrin - je nach Bedarf - zusätzlich " Colony Stimulating Factor " substituiert werden.

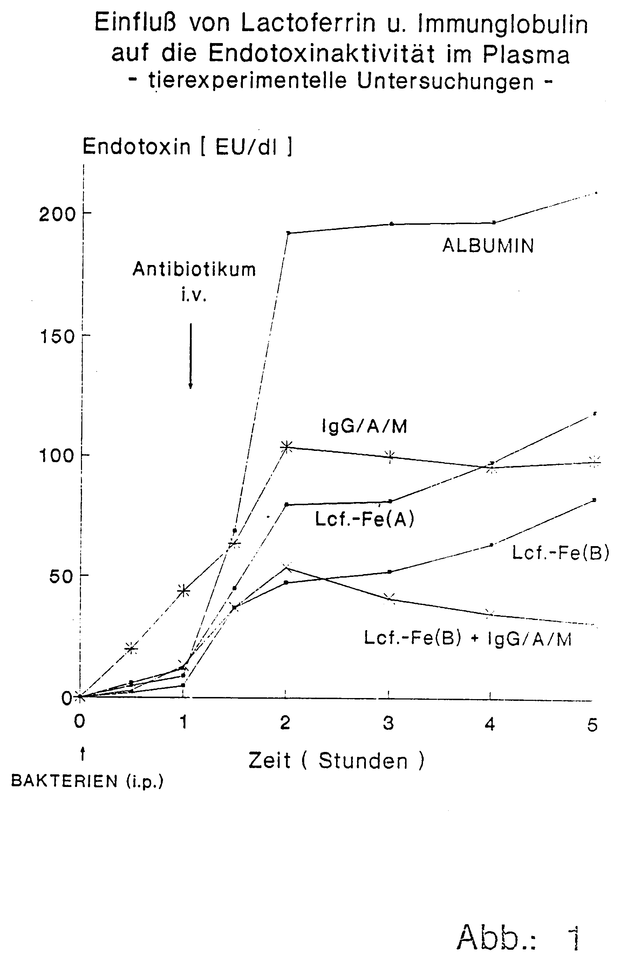

### BEISPIEL: 3 (Abb. 1)

Entsprechend den Bedingungen, wie sie bei der Therapie von gramnegativen Infektionen mit Antibiotika zu finden sind, wurde im Tierversuch ein Endotoxinanstieg im Blut dadurch induziert, daß zunächst Bakterien in die Bauchhöhle gegeben werden und durch die anschließende intravenöse Gabe eines bakteriziden Antibiotikums ein rascher Zerfall dieser Bakterien ausgelöst wird.
Hierzu wurde narkotisierten Wistar-Ratten (250 - 300 g) zur Blutabnahme zunächst ein Katheter in die rechte Halsvene (Vena Jugularis ) eingelegt. Anschließend wurde eine definierte Zahl verschiedener Bakterien ( E.coli, Klebsiella species und Pseudomonas aeruginosa; in einer Konzentration von jeweils 0.75 x 10⁵ cfu/ml/kg ) in die Bauchhöhle appliziert. 30 Minuten nach Gabe der Bakterien in die Bauchhöhle wurde über den Venenkatheter mit einem Perfusor langsam das zu prüfende Präparat ( Lactoferrin sowie Lactoferrin in Kombination mit Immunglobulin) in einer Dosierung von 250 mg/kg Körpergewicht intravenös verabreicht. Die Kontrolltiere erhielten Albumin in entsprechender Dosierung. Zur Erzeugung einer Endotoxinämie wurde eine Stunde nach Bakteriengabe ein bakterizides Antibiotikum (IMIPENEM = Zienam^{R}) intravenös appliziert. Die Blutabnahmen zur Bestimmung der Endotoxinaktivität und der Bakterienzahl erfolgten vor, sowie - in 30 minütigem Abstand - nach der Bakteriengabe über insgesamt 5 Stunden.
Folgende Präparationen wurden i.v. verabreicht: 7-S IgG, angereichert mit 12% IgM(IgG/A/M), Lactoferrin ( Lcf.- Fe(A)) mit einem Eisengehalt von 28 µg Fe³⁺ pro Gramm und Lactoferrin (Lcf.-Fe(B)) mit einem Eisengehalt von 296 µg Fe³⁺ pro Gramm; sowie die Kombination von Lactoferrin (Lcf.-Fe(B)) mit dem IgM(12%)-angereichertem Immunglobulin

### Ergebnisse: ( Abb.: 1)

a) Bei Gabe von Albumin steigt die Endotoxinaktivität im Plasma nach Applikation des Antibiotikums sehr stark an. Bereits eine Stunde nach Gabe des Antibiotikums wird eine mittlere Endotoxinaktivität von 192 EU/dl erreicht. Vier Stunden nach Gabe des Antibiotikums beträgt die Endotoxin aktivität im Plasma 210 EU/dl.
b) Die i.v.-Gabe von dem mit IgM(12%)-angereichertem 7-S IgG(IgG/A/M) führt zu einer Senkung der Endotoxinaktivität im Plasma, die eine Stunde nach Gabe des Antibiotikums, im Vergleich zu der Albumin-Kontrollgruppe, ca. 46 % und vier Stunden später 53% beträgt.
c) Bei i.v.-Gabe von Lactoferrin ( Lcf.-Fe(A) ) wurde die initiale Zunahme der Endotoxinaktivität im Plasma - eine Stunde nach Gabe des Antibiotikums - im Vergleich zur Albumin-Kontrollgruppe um ca 58,5 % reduziert. Vier Stunden nach Gabe des Antibiotikums ist die Endotoxinaktivität bei den Tieren der Lcf.-Fe(A)-Gruppe, noch um ca. 44 % niedriger, als bei der Albumin-Gruppe.
d) Bei i.v.-Gabe von Lactoferrin ( Lcf.-Fe(B) ) beträgt die Reduzierung der initialen Zunahme der Endotoxinaktivität im Plasma - eine Stunde nach Gabe des Antibiotikums - im Vergleich zur Albumin-Kontrollgruppe ca. 75,3 %. Vier Stunden nach Gabe des Antibiotikums ist die Endotoxinaktivität bei der Gruppe, die Lcf.-Fe(B) erhalten hat, noch um ca. 60,5 % niedriger, als bei der Albumin-Gruppe.
e) Bei i.v.-Gabe von Lactoferrin ( Lcf.-Fe(B) ) in Kombination mit einem Immunglobulin, welches mit 12% IgM angereichert ist, ist die initiale Zunahme der Endotoxinaktivität im Plasma - eine Stunde nach Gabe des Antibiotikums - im Vergleich zur Albumin-Kontrollgruppe ca. 73 % niedriger. Vier Stunden nach Gabe des Antibiotikums ist die Endotoxinaktivität bei der Gruppe, welche diese Kombination erhalten hat, ca. 85 % niedriger, als bei der Albumin-Gruppe.

Die Steigerung der Inaktivierungskapazität durch die Kombination von Lactoferrin mit Immunglobulin bietet die Möglichkeit, auch bei einem längeren Endotoxineinstrom einen stärkeren Anstieg der Endotoxinaktivität im Plasma ohne eine übermäßig große Zufuhr von Eisen zu beherrschen. Die alleinige. Gabe von eisenhaltigem Lactoferrin (LcF-Fe(B)) führt bei geringerem Eisengehalt ebenfalls initial zu einer ähnlich starken Reduzierung der Endotoxinaktivität, wie die Kombination von Lactoferrin mit Immunglobulin ( Abb.:1). Ohne Erhöhung der Dosis oder des Eisengehaltes reicht jedoch die Inaktivierungskapazität bei alleiniger Gabe von Lactoferrin mit niedrigem Eisengehalt bei einem fortgesetzten Endotoxinübertritt in das Blut nach einer gewissen Zeit nicht mehr aus, wodurch es wieder zu einem Ansteigen der Endotoxinaktivität im Plasma kommen kann. Die Kombination von Immunglobulin mit Lactoferrin stellt hier, auch bei Verwendung von Lactoferrin mit einem Eisengehalt unter 500 µg/Gramm Lactoferrin eine therapeutisch sehr effiziente Alternative dar.

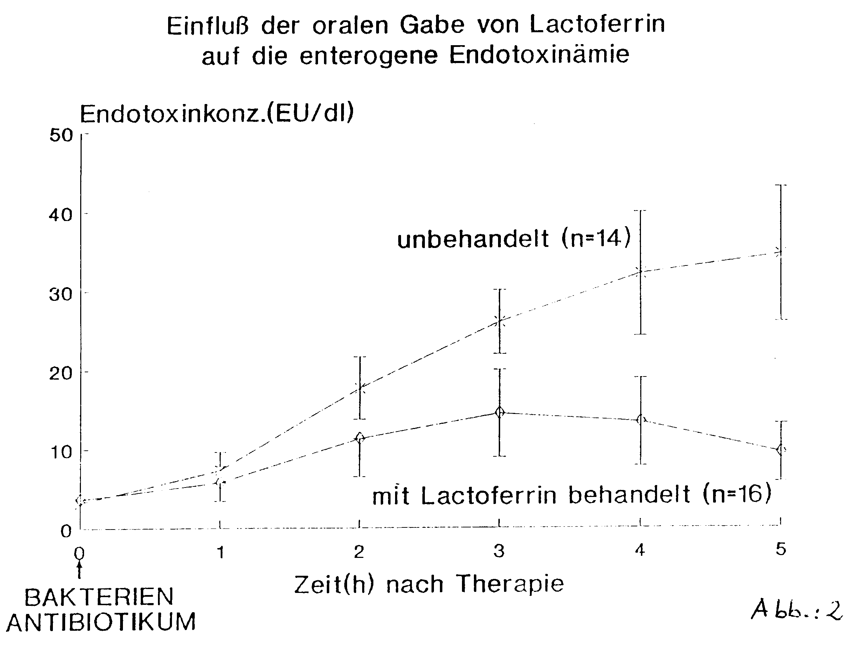

### BEISPIEL: 4 (Abb. 2)

Tierexperimentelle Untersuchungen haben ergeben, daß die Resorptionsrate für Makromoleküle bei Immunsuppression mit Ciclosporin um das 20-fache zunehmen kann. Hierbei kommt es auch zu einer sehr starken Zunahme des Übertritts von Endotoxin aus dem Darmlumen in die Blutbahn [ Nitsche D, Stehle M, Hamelmann H. Der Einfluß der Immunsuppression mit Ciclosporin auf die enterogene Endotoxinämie: Tierexperimentelle Untersuchungen. Langenbecks Archiv für Chirurgie 1990̸ [Suppl.] ]. Daher wurde mit Hilfe dieses Tiermodells geprüft, inwieweit der Übertritt von Endotoxin aus dem Intestinaltrakt in die Blutbahn durch die enterale Behandlung mit Lactoferrin beeinflußt werden kann. Für diese Untersuchungen wurden Wistar-Ratten zunächst 4 Tage lang mit Ciclosporin (12 mg/kg/Tag;i.m.) behandelt. Nach einer Nahrungskarenz von 12 Stunden wurde in Chloralhydrat-Narkose eine Laparotomie vorgenommen und eine Ernährungssonde, die über den Rachenraum eingelegt worden war, in das Duodenum vorgeschoben. Die Tiere erhielten anschließend über diese Sonde eine Suspension von Coli-Bakterien in einer Dosierung von 2,5 x 10^{10̸} cfu/ml pro kg Körpergewicht.

Im Anschluß daran wurde ein nicht resorbierbares Antibiotikum über die Sonde verabreicht, wobei ein Gemisch aus Neomycinsulfat, in einer Dosierung von 16250 I.E. pro kg Kgw, und Bacitracin, in einer Dosierung von 1250 I.E. pro kg Kgw, verwendet wurde. Eine Kontrollgruppe (n = 14 ) erhielt nach Gabe des Antibiotikums über die Sonde eine 2,5 % Albuminlösung in einer Dosierung von 30̸0̸ mg/kg Körpergewicht. Die Therapiegruppe (n=16) erhielt unmittelbar im Anschluß an die Gabe des Antibiotikums eine 2,5 % Lactoferrinlösung ( Lactoferrin bovin ) in einer Dosierung von 30̸0̸ mg/kg über die Sonde. Der Eisengehalt betrug 845 µg Fe³⁺ pro Gramm Lactoferrin.
Zur Bestimmung der Plasma-Endotoxin-Konzentration wurden nach Legen der Duodenalsonde über einen Beobachtungszeitraum von insgesamt 5 Stunden jeweils stündlich 0,2 ml Blut aus der Vena Jugularis entnommen. Die Blutabnahme für die Bestimmung des Nullwertes erfolgte vor der Laparotomie und Bakteriengabe. Die quantitative Endotoxinbestimmung wurde mit einer Modifikation des Limulus-Testes unter Verwendung eines chromogenen Substrates durchgeführt. Die Bestimmung des Ciclosporingehaltes im Blut erfolgte mit einem Fluoreszenz-Assay.

Die mittlere Ciclosporin-Konzentration im Plasma der Tiere betrug bei Versuchsbeginn 1181 ng/ml. Bei sämtlichen Tieren, die mit Ciclosporin vorbehandelt waren, d.h. bei den Tieren beider Gruppen, wurde bereits bei Versuchsbeginn ( O-Wert ) eine geringgradige Endotoxinaktivität im Plasma von 3,2 ± 2,7 EU/dl gemessen, was eine direkte Folge der Ciclosporinvorbehandlung ist. Die Gabe des Antibiotikums über die Sonde führte sowohl bei den Tieren der Albumin-Kontrollgruppe, wie auch bei den Tieren der Lactoferringruppe zu einem Anstieg der Endotoxinaktivität im Plasma, der bereits 30 Minuten später einsetzte. Eine Stunde nach Gabe des Antibiotikums bestand zwischen den Plasmaendotoxinkonzentrationen der beiden Gruppen kein signifikanter Unterschied. Bereits zwei Stunden nach Gabe des Antibiotikums war jedoch die mittlere Plasmaendotoxinkonzentration ( 18,7 ± 3,95 EU/dl ) bei den Tieren, die Albumin erhalten hatten, signifikant ( p = 0.041 ) größer, als bei der Gruppe, die Lactoferrin über die Sonde bekommen hatte. Bei der Lactoferringruppe betrug sie zu diesem Zeitpunkt 11,27 ± 5,76 EU/dl. Während die mittlere Plasmaendotoxinkonzentration der Albumin-Gruppe kontinuierlich weiter anstieg und am Ende des Beobachtungszeitraumes einen Wert von 34,5 ± 8,52 EU/dl erreicht hatte, wurde bei der Lactoferrin-Gruppe im weiteren Verlauf keine signifikante Zunahme der Plasma-Endotoxinaktivität gefunden. Am Ende des Beobachtungszeitraumes, d.h. 5 Stunden nach Gabe des Antibiotikums, betrug die mittlere Plasmaendotoxinkonzentration bei der Lactoferringruppe 9,48 ± 3,64 EU/dl. Zu diesem Zeitpunkt war der Unterschied zur Albumingruppe hochsignifikant ( p < 0.001 ).

Der Verlauf der mittleren Plasmaendotoxinkonzentration bei den Tieren, die über die Sonde Lactoferrin erhalten hatten, der von der zweiten Stunde nach Antibiotikagabe an bis zum Ende des Beobachtungszeitraumes signifikant niedriger war, als bei der Albumin-Kontrollgruppe, beweist, daß durch die enterale Zufuhr von Lactoferrin eine therapeutisch relevante Inaktivierung des Endotoxins im Darmlumen erreicht werden kann. Durch diese Reduzierung der enterogenen Komponente der Endotoxinämie werden gleichzeitig auch die körpereigenen Mechanismen zur Endotoxinneutralisation und Endotoxinelimination entlastet. Damit eignet sich die enterale Gabe von Lactoferrin, per os oder über eine Sonde verabreicht, als Zusatztherapeutikum zur Bekämpfung der Endotoxinämie:
a) bei Patienten mit einer Sepsis, da durch die enterale Applikationsweise, die zusätzlich zur systemischen Behandlung mit einem Endotoxininaktivator erfolgen sollte, die enterogene Komponente der Endotoxinämie beeinflußt werden kann;
b) bei Patienten mit enterogenen Endotoxinämien aufgrund von Permeabilitätsstörungen des Intestinaltraktes, wie sie beispielsweise beim Neugeborenen auftreten können oder wie sie auch bei einer immunsuppressiven Behandlung mit Ciclosporin zu erwarten sind;
c) bei Patienten mit enterogenen Endotoxinämien aufgrund eines gesteigerten Übertrittes von Endotoxin aus dem Darm, wie er bei entzündlichen Darmerkrankungen, z.B. bei der Colitis ulcerosa sowie beim Morbus Crohn und bei anderen entzündlichen Enteritiden auftreten kann;
d) bei Patienten mit enterogenen Endotoxinämien als Folge eines gesteigerten Übertrittes von Endotoxin aus dem Darm, aufgrund eines verstärkten Bakterienzerfalls, wie er im Rahmen einer Therapie mit bakteriziden Antibiotika auftreten kann.
e) bei Patienten mit Störungen der Endotoxinelimination über die Leber, wie sie z.B. bei den Reifungsstörungen der Leber des Neugeborenen, sowie bei allen Leberparenchymschäden, beim Verschlußikterus und bei Störungen der Funktion des RES gefunden werden können.

## Patentansprüche

1. Verwendung eines Mittels, bestehend aus Lactoferrin, an welches Metallionen angelagert sind, zur Herstellung eines Mittels zur Bekämpfung der toxischen Wirkung des Endotoxins.

2. Verwendung eines Mittels nach Anspruch 1, bestehend aus Lactoferrin in Kombination mit Immunglobulinen.

3. Verwendung eines Mittels nach einem der vorangehenden Ansprüche, dadurch gekennzeichnet, daß es sich bei den angelagerten Metallionen um Ionen aus der Gruppe Eisen, Zink, Kupfer, Kobalt, Mangan oder Magnesium handelt.

4. Verwendung eines Mittels nach einem der vorangehenden Ansprüche, dadurch gekennzeichnet, daß es sich um ein Metallion Eisen handelt.

5. Verwendung eines Mittels nach einem der vorangehenden Ansprüche, dadurch gekennzeichnet, daß der Eisengehalt des Lactoferrins mindestens 0,1 µg pro Gramm Lactoferrin beträgt.

6. Verwendung eines Mittels nach einem der vorangehenden Ansprüche 2 bis 5, dadurch gekennzeichnet, daß die Immunglobulinlösungen IgG und IgM oder IgA enthalten.

7. Verwendung eines Mittels nach einem der vorangehenden Ansprüche, dadurch gekennzeichnet, daß das Mittel mit dem Colony Stimulating Factor kombiniert wird.

8. Verwendung eines Mittels nach einem der vorangehenden Ansprüche, dadurch gekennzeichnet, daß das Lactoferrin zur Einbringung per os formuliert ist.

9. Verwendung eines Mittels nach einem der vorangehenden Ansprüche, dadurch gekennzeichnet, daß das Lactoferrin zur direkten Einbringung über eine Sonde in den Intestinaltrakt formuliert ist.

## Claims

1. Use of a substance consisting of lactoferrin which has bound metal ions, for the manufacture of a substance to combat the toxic effects of endotoxins.

2. Use of a substance according to claim 1, consisting of lactoferrin in combination with immunoglobulins.

3. Use of a substance according to one of the preceding claims, characterised in that the metal ions bound are ions out of the group of iron, zinc, copper, cobalt, manganese or magnesium.

4. Use of a substance according to one of the preceding claims, characterised in that the metal ion is iron.

5. Use of a substance according to one of the preceding claims, characterised in that the iron content of the lactoferrin is at least 0,1 µg / g lactoferrin.

6. Use of a substance according to one of the preceding claims 2 to 5, characterised in that the solution of immunoglobulin contains IgG and IgM or IgA.

7. Use of a substance according to one of the preceding claims, characterised in that the substance is combined with the Colony Stimulating Factor.

8. Use of a substance according to one of the preceding claims, characterised in that the lactoferrin is formulated for oral administration.

9. Use of a substance according to one of the preceding claims, characterised in that the lactoferrin is formulated for direct administration by a tube to the gut.

## Revendications

1. Utilisation d'un agent se composant des lactoferrines ayant lié ions des métalliques pour la production d'un agent destiné à combattre les effets toxiques de l'endotoxine.

2. Utilisation selon la revendication 1 se composant des lactoferrines en combinaison avec des immunglobulines.

3. Utilisation d'un agent selon une des revendications précédentes, caracterisée en ce que les ions métalliques lié sont ions de la groupe de fer, zinc, cuivre, cobalt, manganèse ou magnésium.

4. Utilisation d'un agent selon un des revendications précédentes, caractérisée par un ion métallique de fer.

5. Utilisation d'un agent selon une des revendications précédentes caractérisée par le contenu de fer de lactoferrine et au moins 0,1 µg/g lactoferrine.

6. Utilisation d'un agent selon une des revendications 1 à 5, caractérisée par la solution d'immunglobuline contenant IgG et IgM ou IgA.

7. Utilisation d'un agent selon une des revendications précédentes, caractérisée par la combinaison de l'agent avec le Colony Stimulating Factor.

8. Utilisation d'un agent selon une des revendications précédentes, caractérisée par le fait que la lactoferrine est formulée par os pour l'introduction.

9. Utilisation d'un agent selon une des revendications précédentes, caractérisée par le fait que la lactoferrine est formulée pour l'introduction directe dans la voie intestinale par une sonde.
